# EUROPEAN PATENT SPECIFICATION

(11) **EP 1 360 285 B1**
(45) Date of publication and mention of the grant of the patent: **15.04.2009**
(21) Application number: 02711535.1
(22) Date of filing: 01.02.2002
(51) Int. Cl.: C12N 9/96, C12N 9/20

(54) **A METHOD OF PREPARING CROSS-LINKED ENZYME AGGREGATES**
VERFAHREN ZUR HERSTELLUNG VON VERNETZTEN ENZYMAGGREGATEN
TECHNIQUE DE PREPARATION D'AGREGATS D'ENZYMES RETICULEES

(30) Priority: 01.02.2001 NL 1017258
(43) Date of publication of application: 12.11.2003
(73) Proprietor: Technische Universiteit Delft, 2628 BL Delft (NL)
(72) Inventor: LOPEZ SERRANO, Paloma, NL-2623 HA Delft (NL); CAO, Linqiu, NL-2624 PP Delft (NL); VAN RANTWIJK, Frederik, NL-2742 DL Waddinxveen (NL); SHELDON, Roger, Arthur, NL-2281 VA Rijswijk (NL)
(74) Representative: Kupecz, Arpad
(86) International application number: PCT/NL2002/000079
(87) International publication number: WO 2002/061067

(56) References cited:
- EP-A- 1 088 887
- WO-A-95/17504
- US-A- 4 665 028
- CAO L ET AL: "Cross-linked aggregates of penicillin acylase: Robust catalysts for the synthesis of beta-lactam antibiotics." JOURNAL OF MOLECULAR CATALYSIS B ENZYMATIC, vol. 11, no. 4-6, 2001, pages 665-670, XP001079507 ISSN: 1381-1177
- CAO L ET AL: "Cross-linked enzyme aggregates: a simple and effective method for the immobilization of penicillin acylase." ORGANIC LETTERS. UNITED STATES 18 MAY 2000, vol. 2, no. 10, 18 May 2000 (2000-05-18), pages 1361-1364, XP002202388 ISSN: 1523-7060 cited in the application
- VAN UNEN, DIRK-JAN ET AL: "Crown ether activation of crosslinked subtilisin Carlsberg crystals in organic solvents" JOURNAL OF THE CHEMICAL SOCIETY, PERKIN TRANSACTIONS 1: ORGANIC AND BIO-ORGANIC CHEMISTRY (20) , 1998, pages 3341-3343, XP002202482
- KHALAF ET AL.: "Cross-linked enzyme crystals as highly active catalysts in organic solvents" J. AM. CHEM. SOC., vol. 118, 1996, pages 5494-5495, XP002202483
- SHARON C ET AL: "Purification and characterization of a lipase from Pseudomonas aeruginosa KKA-5 and its role in castor oil hydrolysis." JOURNAL OF INDUSTRIAL MICROBIOLOGY & BIOTECHNOLOGY, vol. 20, no. 5, May 1998 (1998-05), pages 304-307, XP001083606 ISSN: 1367-5435
- DATABASE WPI Week 199803 Derwent Publications Ltd., London, GB; AN 1998-021952 XP002202391 & JP 08 113593 A ((UMEM-I) UMEMOTO M), 7 May 1996 (1996-05-07)

## Description

The present invention relates to a method of preparing cross-linked enzyme aggregates comprising the steps of
a) precipitating a dissolved enzyme present in a liquid medium;
b) cross-linking with a cross-linker, and
c) optionally, washing the cross-linked enzyme aggregate

Such a method is generally known in the art. It is used to prepare enzyme preparations for performing various enzyme-catalysed reactions. For example, Cao L. et al. (Organic Letters vol. 2 (10) p. 1361-1364, 2000) describe the preparation of penicilin acylase-based aggregates. The cross-linked aggregates disclosed are less active than the native enzyme.

The object of the present invention is to provide a method making it possible to achieve aggregates with increased enzymatic activity.

To this end, the invention is characterized in that at least one treatment is performed chosen from the group consisting of I) performing step a) in the presence of a compound selected from the group consisting of i) a crown ether, and ii) a surfactant, and II) performing step c) with a second liquid differing in organic composition from the liquid.

Applicants have surprisingly found that such a treatment may result in an cross-linked enzyme aggregate (CLEA) with an enhanced activity. While the present invention does neither guarantee this enhanced activity for a particular enzyme used to catalyse a particular reaction, nor allows for a prediction of an enhanced activity, it does provide for an extra parameter to be controlled to achieve a better aggregate. That is, for a particular enzyme, it may be investigated with little effort and using simple and routine experimentation whether its catalytic activity can be enhanced using the treatment. From a range of enzymes capable of catalyzing the desired reaction, the best CLEA can easily and with little effort be selected. Because the CLEA is insoluble, it can be washed with a second liquid which is completely aqueous, organic, or a mixture of the two. After the treatment, the CLEA may be lyophilized or stored as a suspension for future use.

Overbeeke P.L.A. et al. Describe in J. of Mol. Cat. B: Enzymatic 10, p. 385-393 (2000) a method in which a lipase is crystallized under the influence of PEG in the presence of 2-methyl-2,4-pentanediol, and crystals formed are cross-linked. In contrast, the present invention makes use of a precipitating agent to form aggregates instead of crystals.

CAO L ET AL. describe cross-linked aggregates of penicillin acylase: Robust catalysts for the synthesis of beta-lactam antibiotics". JOURNAL OF MOLECULAR CATALYSIS B ENZYMATIC, vol. 11, no. 4-6, 2001, pages 665-670, XP001079507 ISSN: 1381-1177 .

Further VAN UNEN, DIRK-JAN ET AL. relate to "Crown ether activation of cross-linked subtilisin Carlsberg crystals in organic solvents"JOURNAL OF THE CHEMICAL SOCIETY, PERKIN TRANSACTIONS 1: ORGANIC AND BIO-ORGANIC CHEMISTRY (20), 1998, pages 3341-3343, XP002202482.

Finally, KHALAF ET AL. disclose "Cross-linked enzyme crystals as highly active catalysts in organic solvents" J. AM. CHEM. SOC., vol. 118, 1996, pages 5494-5495, XP002202483.

Preferable, step a) and step b) are performed simultaneously.

This allows for a simplified procedure, requiring less time and effort.

According to a preferred embodiment, the compound is removed after cross-linking.

It is not necessary that the surfactant and or crown ether are present during the catalytic reaction to be performed by the CLEA to maintain the high activity. Thus the compound may be removed, in case it may interfere with the reaction to be catalysed, or for other reasons. Removal may be achieved using any method known in the art, and in particular through dialysis or washing using centrifugation.

According to an important application of the method according to the invention, the enzyme is a lipase.

Hence, the invention also relates to a method for performing a lipase-catalysed reaction, said reaction being chosen from the group consisting of i) transesterification; ii) interesterification; iii) hydrolysis; iv) esterification; v) ammoniolysis; vi) aminolysis; and vii) perhydrolysis, which method is characterized, in that a cross-linked lipase-aggregate is prepared according to the invention, and said cross-linked lipase-aggregate is contacted with a substrate.

The present invention will now be illustrated with reference to the following examples.

### EXAMPLE 1.

### Preparation of cross-linked lipases

- *R. miehei* lipase Lipozyme (Novo Nordisk)
- *Candida antarctica* lipase B SP525 (Novo Nordisk)
- *P. alcaligenes* lipase (Gist-brocades).

### METHOD Ia) In the presence of (NH₄)₂SO₄ (Water)

0.5 ml stock enzyme solution (Lipozyme or SP525, Novo Nordisk, Copenhagen, Denmark) or 50 mg PaL enzyme powder (PaL is *P. alcaligenes* lipase (Gist-Brocades, Delft, the Netherlands)), are dissolved in 1 ml potassium phosphate buffer (100 mM, pH 7) in a 10 ml centrifuge tube. 550 mg (NH₄)₂SO₄ was added followed by 1 ml of a precipitating solution A consisting of 550 mg (NH₄)₂SO₄ (the precipitant) in potassium phosphate buffer (100 mM, pH 7), and 80 µl glutardialdehyde (25% solution in water) are added. The mixture is left stirring at 4°C (room temperature for SP525) for 17 hours. 3 ml H₂O is added to the mixture and then centrifuged. The supernatant is decanted and the residue is washed, centrifuged and decanted 3 more times with H₂O (5 ml each time). The final enzyme preparation is kept in 5 ml H₂O. If necessary the solid is dispersed by stirring with magnets.

*METHOD Ib and Ic) In the presence* of *(NH₄)₂SO₄ and surfactant* The method Ia) was repeated, with the difference that the precipitating solution A contained in addition 25 mg sodium dodecylsulphate (SDS) (method Ib) or 25 mg Triton X-100 (TR) (method Ic).

### METHOD Id) In the presence of dimethoxyethane (DME)

0.5 ml stock enzyme solution (Lipozyme or SP525) or 50 mg PaL enzyme powder, are introduced in a 10 ml centrifuge tube, after which 1 ml potassium phosphate buffer (100 mM, pH 7), 3 ml DME and 80 µl glutardialdehyde are added. The mixture is left stirring at 4°C (room temperature for SP525) for 17 hours.
1 ml DME is added to the mixture and then centrifuged. The supernatant is decantated and the residue is washed, centrifuged and decanted 3 more times with DME (5 ml each time).
The final enzyme preparation is kept in 5 ml DME. If necessary the solid is dispersed by stirring with magnets.

### METHOD Ie) In the presence of DME and a Crownether

Method Id) was repeated, except that an amount of 6.9 mg the crownether dibenzo-18-crown-6 was added too together with the 3 ml DME.
Cross-linked lipase preparations were kept in suspension, either in water or DME, named preparations Ia-e.

### EXAMPLE 2

### Activity tests

The activity of CLEA's prepared according to the preparations Ia-e) were tested with three reactions. These reactions were performed either in an aqueous solution, or in an organic solution.

For tests IIb and IIc, the following general procedure was followed, before performing the assay:
- Of aqueous suspensions, 0.5 ml samples of CLEA suspension were lyophilized in 2 ml Eppendorf cup each.
- Of organic suspensions, 0.5 ml samples of CLEA suspension were centrifuged in a 2 ml Eppendorf cup each, and the supernatant was decanted.
- Native enzyme, used as a control, was used directly from the stock solution (50 µl), or as powder (5 mg), without further treatment.

For text IIb and IIc, analyses were performed using a GC Varian Star 3600 gas chromatograph (Varian). Column: CP WAX 52 CB 50 m x 0.53 mm. Carrier gas: nitrogen.

### Test IIa) Hydrolysis of p-nitrophenyl propionate (performed in water)

The enzyme preparation (50 µl) was suspended in 0.5 ml water (1.5 ml in case of SP525) and 200 µl (50 µl in case of SP525) of the suspension was added to 2.5 ml ester solution (0.4 mM p-nitrophenyl propionate in water at 25°C). Measurement of the increase of absorbance at 348 nm (appearance of p-nitrophenol) in the hydrolysis of p-nitrophenyl propionate was monitored using a Cary 3-Bio UV spectrophotometer from Varian. Values are given as percentage of the activity of the native enzyme.

The result is summarized in table 1

**Table 1**

| Hydrolysis of p-nitrophenyl propionate in water | | | | | | |
|---|---|---|---|---|---|---|
| **Enz** | **Ia** | **Ib** | **Ic** | **Id** | **Ie** | **Native** |
| **Lipozyme** | 152 | 218 | 44 | 0 | 0 | 100 |
| **SP525** | 93 | 40 | 129 | 151 | 177 | 100 |

| | | | | | | |
|---|---|---|---|---|---|---|
| % of activity of native enzyme | | | | | | |

### Test IIb) Transesterification of ethyl octanoate with octanol (performed in diisopropylether)

0.5 ml diisopropyl ether is added to the Eppendorf cup containing the enzyme. 50 mg activated zeolite NaA (Aldrich) is added. 0.5 ml solution of 86 mM ethyl octanoate, 400 mM octanol, and 50 mM decane in diisopropyl ether is added and left stirring at room temperature. At the specified time, the reaction mixture is centrifuged, an aliquot (100 µl) withdrawn, dissolved in 0.5 ml DME, and analyzed by GC.

The result is summarized in table 2

**Table 2**

| Transesterification of ethyl octanoate with octanol in i-Pr₂O | | | | | | |
|---|---|---|---|---|---|---|
| **Enz** | **Ia** | **Id** | **Ic** | **Id** | **Ie** | **Native** |
| **Lipozyme** | 0.16 | 3.48 | 0.33 | 23.79 | 18.89 | 0.16 |
| **Pal** | 0.15 | 7.11 | 24.18 | 0.11 | 0.27 | 17.55 |

| | | | | | | |
|---|---|---|---|---|---|---|
| Activity expressed as µmol of octyl octanoate produced in 24 h. | | | | | | |

### Test IIc) Hydrolysis of ethyl octanoate (performed in DME/water mixture)

50 µl H₂O and 0.95 ml solution of 43 mM ethyl octanoate, and 25 mM decane is added to Eppendorf cup containing the enzyme and left stirring at room temperature. At the specified time, anhydrous Na₂SO₄ is added to the reaction mixture, shaken, and centrifuged. An aliquot (100 µl) is then withdrawn, dissolved in 0.5 ml DME, and analyzed by GC.

The result is summarized in table 3

**Table 3**

| Hydrolysis of ethyl octanoate in DME/water | | | | | | |
|---|---|---|---|---|---|---|
| **Enz** | **Ia** | **Ib** | **Ic** | **Id** | **Ie** | **Native** |
| **Lipozyme 24h** | 24.34 | 40.48 | 25.37 | 22.85 | 22.73 | 16.34 |
| **Pal. 3h** | 35.82 | 32.02 | 40.26 | 0.49 | 0.37 | 3.34 |

| | | | | | | |
|---|---|---|---|---|---|---|
| Activity expressed as µmol of octanoic acid produced in 24 or 3 h. | | | | | | |

As shown by the above results, adding a surfactant or a crown ether in accordance with the present invention, may have a beneficial effect on the enzymatic activity.

### EXAMPLE 3

### Washing test

Methode Ia was repeated using SP525. The supernatant is decanted and the pellet resuspended in 5 ml water, centrifuged and the supernatant is decanted.

The pellet obtained is subjected to the same washing procedure with 3 * 5 ml water or DME. After the last washing step, the CLEA is kept in 5 ml of the same solvent.

The two CLEAs were tested using the transesterification of ethyl octanoate with octanol (as described in test IIc).

0.5 ml DME is added to 50 µl enzyme preparation in a 2 ml Eppendorf. The enzyme washed with water only was previously lyophilized in the Eppendorf cup. 50 mg activated zeolite NaA is added. 0.5 ml solution of 86 mM ethyl octanoate, 400 mM octanol, and 50 mM decane in DME is added and left stirring at room temperature. After 1 hour, the reaction mixture is centrifuged, an aliquot (100 µl) withdrawn, dissolved in 0.5 ml DME, and analyzed by GC.

**Table 4**

| Transesterification of ethyl octanoate with octanol (in DME) | |
|---|---|
| Processing solvent | Activity |
| Water | 5.41 |
| DME | 14.03 |

| | |
|---|---|
| Activity expressed as µmol of octyl octanoate produced in 1 h. | |

### EXAMPLE 4

Lipase CLEAs were prepared according the procedure described in example 1 and were tested in the hydrolysis of methyl mandelate. The enzyme was added to a 50 mM solution of methyl mandelate in dimethoxyethane-potassium phosphate buffer pH 7 (95:5, v/v). After 48 hours the conversion and enantiomeric excess of the substrate (eeₛ) were analyzed using HPLC (see Table 5).

**Table 5**

| Precipitant | Additive | Conv. (%) | eeₛ | E |
|---|---|---|---|---|
| Dimethoxyethane | - | 31.0 | 0.31 (R) | 7 |
| Dimethoxyethane | CR | 33.5 | 0.33 (R) | 7 |
| Acetone | - | 26.7 | 0.26 (R) | 8 |
| (NH₄)₂SO₄ | SDS | 27.9 | 0.27 (R) | 8 |
| (NH₄) ₂SO₄ | TR | 23.2 | 0.24 (R) | 10 |
| Free enzyme | - | 33.9 | 0.33 (R) | 6 |

| | | | | |
|---|---|---|---|---|
| CR : Crownether dibenzo-18-crown-6 SDS: Sodium dodecylsulfate TR : Triton X-100 eeₛ : enantiomeric excess of the substrate E : Enantiomeric ratio. | | | | |

From table 5 it can be seen that the Enantiomeric ratio can be increased using the method according to the present invention.

## Claims

1. Method of preparing cross-linked enzyme aggregates comprising the steps of
a) precipitating a dissolved enzyme present in a liquid medium; and
b) cross-linking with a cross-linker,
**characterized in that**,
step a) is performed in the presence of a compound selected from a crown ether or a surfactant.

2. The method according to claim 1, **characterized in that** the cross-linked enzyme aggregate is washed with a second liquid different in organic composition from the liquid used in step a).

3. Method according to claim 1, **characterized, in that** steps a) and b) are performed simultaneously.

4. Method according to any of the preceding claims, **characterized, in that** the compound is removed after cross-linking.

5. Method according to any of the preceding claims, **characterized, in that** the enzyme is a lipase.

6. Method for performing a lipase-catalysed reaction, said reaction being chosen from the group consisting of i) transesterification; ii) hydrolysis; and iii) ammoniolysis, **characterized, in that** a cross-linked lipase-aggregate is prepared according to claim 5, and said cross-linked lipase-aggregate is contacted with a substrate.

## Patentansprüche

1. Verfahren zur Herstellung von vernetzten Enzymaggregaten, dass die Schritte umfasst:
a) Ausfällen eines gelösten Enzyms, das in einem flüssigen Medium vorliegt; und
b) Vernetzen mit einem Vernetzungsmittel,
**dadurch gekennzeichnet, dass**
Schritt a) in Gegenwart einer Verbindung, die aus einem Kronenether oder einem Tensid ausgewählt wird, durchgeführt wird.

2. Das Verfahren gemäß Anspruch 1, **dadurch gekennzeichnet, dass** das vernetzte Enzymaggregat mit einer zweiten Flüssigkeit gewaschen wird, die in ihrer organischen Zusammensetzung von der in Schritt a) verwendeten Flüssigkeit unterschiedlich ist.

3. Verfahren gemäß Anspruch 1, **dadurch gekennzeichnet, dass** die Schritte a) und b) gleichzeitig ausgeführt werden.

4. Verfahren gemäß einem der vorangegangenen Ansprüche, **dadurch gekennzeichnet, dass** die Verbindung nach dem Vernetzen entfernt wird.

5. Verfahren gemäß einem der vorangegangenen Ansprüche, **dadurch gekennzeichnet, dass** das Enzym eine Lipase ist.

6. Verfahren zur Durchführung einer Lipase-katalysierten Reaktion, wobei die Reaktion aus der Gruppe, die aus i) Transveresterung; ii) Hydrolyse; und iii) Ammonolyse besteht, ausgewählt wird, **dadurch gekennzeichnet, dass** ein vernetztes Lipase-Aggregat gemäß Anspruch 5 hergestellt wird und das vernetzte Lipase-Aggregat mit einem Substrat kontaktiert wird.

## Revendications

1. Procédé de préparation d'agrégats d'enzymes réticulés comprenant les étapes de
a) précipitation d'un enzyme dissous présent dans un milieu liquide ; et
b) réticulation avec un agent de réticulation,
**caractérisé en ce que**,
l'étape a) est réalisée en présence d'un composé choisi parmi un éther couronne ou un tensioactif.

2. Procédé selon la revendication 1, **caractérisé en ce que** l'agrégat d'enzymes réticulés est lavé avec un deuxième liquide de composition organique différente du liquide utilisé dans l'étape a).

3. Procédé selon la revendication 1, **caractérisé en ce que** les étapes a) et b) sont réalisées simultanément.

4. Procédé selon l'une quelconque des revendications précédentes, **caractérisé en ce que** le composé est éliminé après réticulation.

5. Procédé selon l'une quelconque des revendications précédentes, **caractérisé en ce que** l'enzyme est une lipase.

6. Procédé pour réaliser une réaction catalysée par une lipase, ladite réaction étant choisie parmi le groupe consistant en i) transestérification ; ii) hydrolyse ; et iii) ammoniolyse, **caractérisé en ce que** un agrégat réticulé par une lipase est préparé selon la revendication 5, et ledit agrégat réticulé par une lipase est mis en contact avec un substrat.
